# EUROPEAN PATENT SPECIFICATION

(11) **EP 2 155 158 B1**
(45) Date of publication and mention of the grant of the patent: **21.08.2019**
(21) Application number: 08762318.7
(22) Date of filing: 10.06.2008
(51) Int. Cl.: A61K 9/00, A61K 8/42, A61Q 17/00, A61Q 19/00

(54) **PERSONAL LUBRICANT COMPOSITION**
GLEITMITTELZUSAMMENSETZUNG
COMPOSITION LUBRIFIANTE À USAGE PERSONNEL

(30) Priority: 15.06.2007 GB 0711766
(43) Date of publication of application: 24.02.2010
(73) Proprietor: LRC Products Limited, Slough, Berkshire SL1 3UH (GB)
(72) Inventor: MCLEAY, Laura, London EC4V 6BW (GB)
(74) Representative: Carlin, Robert George
(86) International application number: PCT/GB2008/001981
(87) International publication number: WO 2008/152374

(56) References cited:
- US-A- 4 136 163
- US-A- 5 653 971
- US-A1- 2005 244 520

## Description

The present invention relates generally to lubricants, particularly but not exclusively to personal lubricants, particularly personal lubricants which have a cooling effect in use, and to a method of making them.

Personal lubricants are specialised lubricants which serve to reduce friction with body tissues. In particular, personal lubricants may be used to provide lubrication, or slip, during sexual activity. For example, personal lubricants can be used to increase pleasure or reduce pain during sexual intercourse, and can aid in reducing vaginal dryness. In medicine, personal lubricants may be employed for gynaecological examinations and the like.

A wide variety of personal lubricants are currently available. These lubricants generally function by supplying water on a body surface in a gelled or viscous form, by comprising a water-soluble polymer, such as a water-soluble cellulose derivative, or other water soluble polymers such as polyvinylpyrrolidone, polyvinyl alcohol and the like. In use on a body surface, these gelled or viscous systems retain water on the body surface to which they are applied, and the water provides lubrication. One or more humectants may be added to aid water retention on the body surface, so increasing the level of lubrication (that is, the lubricity of the formulation) provided, and/or increasing the length of time for which lubrication persists (that is, the longevity of lubricity).

In recent years it has become increasingly popular to add various active ingredients to personal lubricant compositions, in order to, for example, enhance pleasurable feelings during sexual activity, and/or to heighten sexual arousal. Personal lubricants comprising such active ingredients are designed to cause physiological or physical changes in the area to which they are applied. For example, personal lubricants may comprise cooling or warming compounds.

WO 2007/136586, for example, relates to personal care compositions comprising polytrimethylene ether glycol. In particular, personal lubricant compositions are disclosed. These compositions have a warming effect. It is taught that the feeling of warmth generated by the compositions is soothing to the skin or mucous membranes where they are applied.

Similarly, US 6,673,844 relates to warming compositions. The compositions comprise one or more warming compounds, typically vanillyl. Cooling agents are also included, and it is reported that the combination of a cooling agent and a warming agent gives a synergistic improved warming effect; there is no teaching as to how to improve cooling sensations. A number of suitable cooling agents are suggested; the most preferred are 3-(1-menthoxy)propane-1,2-diol and 3-(menthoxy)-2-methylpropane-1,2-diol.

In the context of personal lubricants, coolants are thought to improve desirable sensate properties. This is generally explained by the chemical reaction of coolant compounds on the nerve endings responsible for the sensation of coldness, rather than because of a physical drop in temperature at the body surface (such as would be caused by latent heat of evaporation, for example). In particular, personal lubricant compositions comprising cooling compounds, or coolants, exert cooling and tingling sensations in use, and are considered to have stimulating effects, which are thought to improve and/or aid female and male pleasure.

To date, the coolants that have been used in sexual lubricants include menthol, menthone glycerine acetone and menthyl lactate. Menthol is by far the most commonly used coolant in personal lubricants, however. Currently, almost all cooling personal lubricants comprise menthol, often as the sole coolant. However, menthol may be present in combination with other coolants, and these additional coolants are usually menthol- or menthyl-derivatives.

For example, US 6,673,844, discussed above, lists menthol as a suitable coolant; there is no suggestion that the use of menthol should be avoided. WO 2006/092074 relates to compounds having cooling properties, and discloses compounds having particularly strong cooling effects and their use in products that are applied to the mouth or the skin to give a cooling sensation. Example compounds include N-benzooxazol-4-yl 3-p-menthane-carboxamide; N-benzooxazol-6-yl 3-p-menthanecarboxamide, and N-4-(oxazol-5-yl)-phenyl-3-p-menthancarboxamide. Mouthwashes and toothpastes comprising the compounds are disclosed but there is no disclosure of personal lubricants, nor any teaching that these compounds are, in fact, suitable for use in personal lubricants. Furthermore, although these compounds are taught to have a stronger cooling effect than menthol, WO 2006/092074 does not teach or suggest that menthol should be avoided.

Menthol has a number of negative attributes. Depending upon the menthol concentration, the disadvantages of menthol include: a strong minty smell, which some users find unpleasant; bitter taste; potential for causing irritation (including irritation of mucous membranes); potential for burning at higher concentrations; and high volatility (which has led to eye irritation from some aftershave lotions). The high volatility of menthol also results in a loss of coolant from compositions comprising menthol, so that functionality is lost over time. In addition, high concentrations of menthol, which can occur, for example, as a result of menthol separating out from its carrier, can result in localised burning. Many of these disadvantages, such as menthol's potential to cause irritation and burning, are particularly undesirable in the context of personal lubricant compositions, which are generally applied to particularly sensitive areas of the body. Many of these disadvantages have been found to be shared by many of the commonly used "menthoxy" coolants, which are also commonly used in cooling personal lubricant compositions, often in combination with menthol.

Additional disadvantages of menthol include crystallisation at low temperatures. Crystallisation of menthol in personal lubricant formulations results in clouding of the formulation, or even leads to a granulation effect being perceived on application, which many users find undesirable.

We have found that many of the currently marketed products comprise unacceptably high levels of menthol, which levels can be associated with irritation. Whilst decreasing the menthol concentration can reduce levels of irritation, to acceptably low rates, we have found that the very low levels of menthol necessary to minimise irritation also result in reduced menthol functionality. As a consequence, products containing low levels of menthol lack user-perceived benefits such as cooling and tingling sensations and so do not give the desired sensate characteristics in use. Accordingly, the problems associated with menthol cannot be overcome simply by reducing the concentration of menthol used, because products with low levels of menthol have been found to be ineffective.

However, despite its many disadvantages, menthol has long been the coolant of choice in personal lubricant compositions, even in concentrations which may be irritant.

US 2005/244520 A1 discloses topical menthol, or a related cooling compound, to induce lubrication.

We have now recognised that there is a need for alternative cooling personal lubricant compositions, and have devised compositions which minimise the problems seen with the prior art compositions.

The present invention provides the use of an aqueous-compatible sexual lubricant composition to provide lubrication during sexual activity, wherein the composition comprises a coolant which is a cyclic carboxamide. Any suitable cyclic carboxamide that acts as a coolant may be used. Preferably, the cyclic carboxamide is N-ethyl-p-menthane-3-carboxamide.

There is also provided the use of an aqueous-compatible sexual lubricant composition to provide lubrication during sexual activity, wherein the composition comprises a coolant which is a cyclic carboxamide and a second coolant. Preferably the cyclic carboxamide is N- ethyl-p-menthane-3-carboxamide. Any suitable second coolant may be used. Preferred second coolants include menthyl lactate, menthone glycerine acetal, menthoxypropane diol and isopulegol. Most preferably, the personal lubricant composition comprises a cyclic carboxamide which is N-ethyl-p-menthane-3-carboxamide and a second coolant which is menthyl lactate, menthone glycerine acetal, menthoxypropane diol, isopulegol, or any other suitable coolant.

In another embodiment, there is provided the use of an aqueous-compatible sexual lubricant composition to provide lubrication during sexual activity, wherein the composition comprises a coolant which is a cyclic carboxamide, which composition is substantially free from menthol. Preferably, the composition is free from menthol. There is also provided the use of an aqueous-compatible sexual lubricant composition to provide lubrication during sexual activity, wherein the composition comprises a coolant which is a cyclic carboxamide and a second coolant which composition is substantially free from menthol. Preferably, the composition is free from menthol. Any suitable cyclic carboxamide which acts as a coolant may be used. Preferably the cyclic carboxamide is N-ethyl-p-menthane-3-carboxamide. Any suitable second coolant may be used. One or more second coolants may be used. A suitable second coolant is, for example, menthyl lactate, menthone glycerine acetal and menthoxypropane diol or isopulegol. Most preferably, the personal lubricant composition comprises a cyclic carboxamide which is N-ethyl-p-menthane-3-carboxamide and a second coolant which is menthyl lactate, menthone glycerine acetal, menthoxypropane diol or isopulegol, and the composition is substantially free from menthol.

Preferably, the total coolant concentration in the composition is from 0.01 to 1.5% w/w, by weight of the total composition. By "total coolant concentration" we mean the concentration of the cyclic carboxamide and of any second coolant which is present in the composition.

In a preferred embodiment, the composition comprises the cyclic carboxamide at a concentration of 0.016 - 0.020% w/w, by weight of the total composition. In a particularly preferred embodiment, the composition comprises N-ethyl-p-menthane-3-carboxamide at a concentration of 0.016 - 0.020% w/w, by weight of the total composition.

The compositions of the invention are aqueous. Aqueous compositions are particularly preferred because they are compatible with all condom types, including natural rubber latex, synthetic polyisoprene and polyurethane condoms.

Suitably, the composition further comprises one or more thickening polymers. Preferred thickening polymers include, but are not limited to carbomers; celluloses, such as hydroxypropylmethylcellulose and hydroxyethylcellulose; and polyacrylates such as glyceryl polyacrylate. Hydroxyethylcellulose is a particularly preferred thickening polymer.

Suitably, the compositions may further comprise one or more preservatives. Suitable preservatives include antimicrobial preservatives such as parabens, sorbic acid, phenoxyethanol, benzoic acid and sodium benzoate. A particularly preferred preservative is benzoic acid.

Preferably, the compositions further comprise one or more pH adjusters. Suitably, the pH adjuster is selected for compatibility with the thickening polymer. Suitable pH adjusters include, but are not limited to, organic acids and organic bases. For example, preferred pH adjusters include organic acids such as citric acid, and bases such as triethanolamine and aminomethylpropanol.

The compositions optionally further comprise a sweetener. Sodium saccharin is a preferred sweetener.

In a preferred aspect, the present invention also provides the use of an aqueous-compatible sexual lubricant composition to provide lubrication during sexual activity, wherein the composition comprises a solvent; a thickening polymer; a coolant which is N-ethyl-p-menthane-3-carboxamide; and optionally a second coolant, which composition is free from menthol. The optional second coolant may be any suitable coolant, such as, for example, menthyl lactate, menthone glycerine acetal, menthoxypropane diol or isopulegol.

There is also described herein a method of making personal lubricant compositions comprising mixing together the components of the compositions as generally described herein.

There is also described herein a method of making a personal lubricant composition comprising mixing a solvent, a thickening polymer and a coolant which is a cyclic carboxamide.

There is also described herein a method of making a personal lubricant composition, which method comprises mixing a solvent, a thickening polymer, a preservative, a pH adjuster, and a coolant which is a cyclic carboxamide.

Optionally, the method further comprises adding a humectant to the composition. Optionally, the method further comprises adding a second coolant to the composition. Preferably, the optional second coolant is isopulegol. Optionally, the method further comprises adding a sweetener to the composition.

The compositions provided by the present invention are free from menthol. Accordingly, the compositions have significant advantages over currently available cooling lubricants, which almost without exception comprise menthol, because compositions of the invention avoid all the disadvantages inherent in using menthol. The compositions provided herein are substantially non-irritating, non-burning and non-cytotoxic.

N-ethyl-p-menthane-3-carboxamide (WS-3) does not crystallise at low temperatures, and we have found that, in contrast to menthol-containing compositions, N-ethyl-p-menthane-3-carboxamide-containing compositions do not go cloudy after exposure to or storage at low temperatures.

Furthermore, N-ethyl-p-menthane-3-carboxamide has a lower volatility than menthol, so the compositions of the present invention do not suffer from the same loss-of-coolant problems found with menthol-based cooling personal lubricants. Storage of the preferred formulation for 3 months at 40°C showed no loss of N-ethyl-p-menthane-3-carboxamide, whereas storage of a similar formulation containing menthol for 3 months at 40°C showed a significant reduction in the level of retained menthol of about 50%.

The compositions of the invention comprise a coolant which is a cyclic carboxamide in place of menthol, which is currently the coolant of choice in personal lubricant compositions. Optionally, the compositions may further comprise one or more second coolants.

Any suitable cyclic carboxamide which has a cooling effect can be used. As used herein, the term "cyclic carboxamide" refers particularly to cyclic compounds which are based on the menthol cyclic structure (shown below), but with a carbon-carbon (C-C) link at the 3-position on the ring, in place of the carbon-oxygen (C-O) bond which is found in menthol and in the various menthoxy coolants such as menthone glycerine acetal, menthyl lactate and menthoxypropane-1,2-diol.

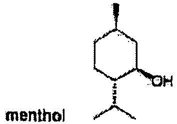

N-ethyl-p-menthane-3-carboxamide (WS-3) is a particularly preferred cyclic carboxamide. 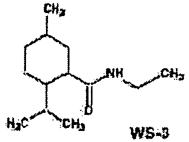 N-ethyl-p-menthane-3-carboxamide

N-ethyl-p-menthane-3-carboxamide has, to date, primarily been used in oral, medicinal and confectionary products, such as breath freshening chewing gums. We have found that N-ethyl-p-menthane-3-carboxamide provides particularly good cooling effects. We have found that N-ethyl-p-menthane-3-carboxamide is generally superior to the coolants which have previously been used in personal lubricant compositions (that is, menthol, menthone glycerine acetal and the like). For example, as indicated in Table 1, N-ethyl-p-menthane-3-carboxamide has been found to have a superior relative cooling strength in comparison to all the menthoxy coolants.

**Table 1**

| Coolant | Structure | Cooling strength relative to menthol |
|---|---|---|
| Menthoxypropane-1,2-diol | Menthoxy | 15.8 - 39.5 |
| Isopulegol | Menthoxy | 25 |
| Menthone lactate | Menthoxy | 41 |
| Menthyl lactate | Menthoxy | 43 |
| WS-23 | Linear carboxamide | 75 |
| Menthol | Menthoxy | 100 |
| WS-3 | Cyclic carboxamide | 150 |

In addition, we have found that N-ethyl-p-menthane-3-carboxamide lacks the volatile side effects of menthol, and provides a smooth, gradual cooling sensation, which has been reported as particularly pleasant by personal lubricant-users.

In preliminary trials, we have found that the compositions have a pleasant lubricant feel, being reported as silky, smooth and lubricious; and give a pleasant sensation during use. The compositions are generally very well received by users in comparison to other available personal lubricants.

Significantly, N-ethyl-p-menthane-3-carboxamide does not have any of the disadvantages of menthol, such as propensity to cause irritation and burning. We have found that personal lubricants comprising N-ethyl-p-menthane-3-carboxamide are essentially non-cytotoxic, and do not cause irritation, itching or burning.

Any suitable amount of the cyclic carboxamide can be used. A preferred amount of cyclic carboxamide is from 0.01 to 1.5% by weight of the formulation, more preferably, from 0.01 to 0.3% by weight. Any suitable amount of N-ethyl-p-menthane-3-carboxamide can be used. A preferred amount of N-ethyl-p-menthane-3-carboxamide is from 0.01 to 1.5% by weight of the formulation. A more preferred amount of N-ethyl-p-menthane-3-carboxamide is from 0.01 to 0.30% by weight. In a particularly preferred embodiment, the compositions comprise N-ethyl-p-menthane-3-carboxamide in an amount from 0.016 to 0.020% by weight of the formulation.

Optionally, the compositions comprise one or more second coolants, in addition to N-ethyl-p-menthane-3-carboxamide. Any suitable second coolant which is not menthol can be used in the compositions. Preferably, the second coolant is a coolant compound which does not have any of the disadvantages of menthol (such as potential for irritation and burning, and so on). Any suitable amount of second coolant may be employed, as will be apparent to the skilled person.

For example, suitable second coolants include, but are not limited to menthyl lactate, menthone glycerine acetal, menthoxypropane diol and isopulegol. By isopulegol, we mean particularly (-)-isopulegol, or 2-isopropenyl-5-methyl-cyclohexanol: 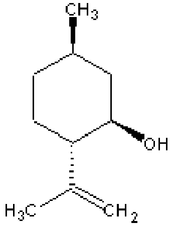 (-)-Isopulegol (Coolact P™).
(-)-Isopulegol is sold under the name Coolact P™ by Takasago International.

Personal lubricant compositions suitably comprise a solvent. Any suitable solvent may be used. Suitable solvents include, but are not limited to, water, glycerine and propylene glycol, alone or in combination. However, the compositions are aqueous compositions. the solvent comprises water, optionally in combination with additional ingredients such as, for example glycerine and/or propylene glycol.

Suitably, the compositions comprise one or more thickening polymers. By "thickening polymer" we mean to include any polymer which serves to increase the viscosity of the composition. Any suitable thickening polymer may be used. For example, suitable thickening polymers include, but are not limited to one or more of carbomers; celluloses such as, for example, hydroxypropylmethylcellulose or hydroxyethylcellulose; and polyacrylates such as glyceryl polyacrylate. Glyceryl polyacrylate is commercially available as a component of, for example, Lubragel™ compositions. Accordingly, the compositions may comprise a thickening polymer which is glyceryl polyacrylate in the form of a Lubragel™ composition. We have
found that Lubragel™ II XD (commercially available from United-Guardian, Inc) provides particularly good lubricity, particularly after dilution. Accordingly, of the commercially available Lubragels™, Lubragel™ II XD is particularly preferred.

Lubragel™ II XD (United-Guardian, Inc) has the following composition:

| Ingredient | Amount (%w/w) |
|---|---|
| Water | 40- 50 |
| Glycerin | 40 - 40 |
| Glyceryl polyacrylate | 1 -5 |
| Methylparaben | 0.045 - 0.055 |
| Propylparaben | 0.027 - 0.033 |

As will be apparent to the skilled person, the formulation may comprise a mixture of two or more thickening polymers. For example, two or more thickening polymers may be employed in combination, to achieve the desired viscosity and lubricity of the composition.

A particularly preferred thickening polymer is hydroxyethylcellulose. A suitable hydroxyethylcellulose is Natrosol™ 250 HHX (commercially available from Hercules, Inc).

The thickening polymer may be included in the composition in any suitable amount. Preferred amounts of thickening polymer include amounts from about 1.0 % w/w to about 3.0 % w/w, more preferably from about 1.5 % w/w to about 2.5 % w/w, most preferably about 2.0 % w/w. In a particularly preferred embodiment, the thickening polymer is about 2.0 % w/w hydroxyethylcellulose.

Optionally, the compositions may further comprise a humectant. Humectants are generally included in personal lubricant compositions in order to increase the lubricity of the composition, and/or to increase the longevity of the lubricity in use, by aiding the retention of water by the thickening polymer. The term "humectant" as used herein includes any compound or substance that, in use, aids water retention by the thickening polymer. Any suitable humectant may be use, as will be apparent to the skilled person. For example, suitable humectants include, but are not limited to, glycerine; polyols such as, for example, sorbitol and mannitol; and glycols such as polyethylene glycol and propylene glycol. A preferred humectant is propylene glycol. Any suitable amount of humectant may be used, as will be apparent to the skilled person in light of conventional personal lubricant formulating practice. Preferably, the humectant is present in an amount from about 5% to about 20% w/w (by weight of the total formulation), more preferably the humectant is present in an amount from about 5% to about 15% w/w, most preferably the humectant is present in an amount of about 10% w/w. In a particularly preferred embodiment, the composition comprises about 10% w/w propylene glycol.

The compositions optionally comprise a pH adjuster. Any suitable pH adjuster may be used. Suitably, the pH adjuster is selected to be compatible with the thickening polymer. Preferred pH adjusters include organic acids and organic bases. For example, preferred pH adjusters include sodium hydroxide, citric acid, triethanolamine and aminomethylpropanol. In a particularly preferred embodiment, the pH adjuster is sodium hydroxide. The pH adjuster is preferably added in an amount to adjust the pH to within a pH range suitable for application to the human genital area. For example, the pH is suitably adjusted to from about pH 3.8 to about pH 4.8.

The compositions suitably further comprise a preservative, such as an antimicrobial and/or antifungal preservative. Any suitable preservative may be used. However, preferred preservatives include parabens, sorbic acid, phenoxyethanol, sodium benzoate and the like. Optionally, mixtures of two or more preservatives may be used. A particularly preferred preservative is benzoic acid. The preservative may be present in any suitable amount. Preferred amounts include from amount 0.1 to about 0.2% by weight of the formulation. A particularly preferred amount is about 0.15%w/w. In a preferred embodiment, the composition comprises 0.10% w/w benzoic acid.

The compositions optionally comprise a sweetener. Any suitable sweetener may be used, in accordance with general formulation practice. Preferred sweeteners include, for example, saccharins, such as sodium saccharin; fructose; and sorbitol. A particularly preferred sweetener is sodium saccharin.

Preferred compositions comprise the following ingredients in amounts within the ranges indicated:

**Table 2**

| Ingredient | Preferred amounts (%w/w) |
|---|---|
| Solvent | 75 - 90 |
| Humectant | 5 - 20 |
| Thickener | 1.5 - 2.5 |
| Cyclic carboxamide | 0.01 - 1.5 |
| pH adjuster | To give final pH from pH 3.8 to pH 4.8 |
| Preservative | 0.1 - 0.2 |
| Sweetener | 0.05 - 0.10 |

Any suitable cyclic carboxamide can be used. Particularly preferred is N-ethyl-p-menthane-3-carboxamide. The solvent may be any suitable solvent. Suitable solvents include water, propylene glycol, and glycerine, alone or in combination. Water is a particularly preferred solvent. Optionally, the solvent may comprise water in combination with propylene glycol and/or glycerine. Any suitable humectant may be used. Preferred humectants include glycerine; polyols such as sorbitol and mannitol; polyethylene glycols; and polypropylene glycols. Any suitable thickener may be used. Preferred thickeners include carbomers; celluloses such as hydroxyethylcellulose; and glycerylpolyacrylate, for example in the form of a Lubragel™ composition. Any suitable pH adjuster may be used, in order to achieve the required final pH of the composition. A suitable pH is from about pH3.8 to about pH4.8. Suitable pH adjusters include organic acids and organic bases. Any suitable preservative can be used, as will be apparent to the skilled person. Preferred preservatives include parabens, sorbic acid, phenoxyethanol, benzoic acid and sodium benzoate. The sweetener may be any suitable sweetener. Suitable sweeteners include, but are not limited to, saccharins such as sodium saccharin, fructose and sorbitol.

The composition optionally comprises one or more second coolants, in addition to the cyclic carboxamide. Any suitable second coolant may be used. However, particularly suitable second coolants include menthyl lactate, menthone glycerine acetal, isopulegol and menthoxypropane diol.

Generally, the compositions of the invention can be made by simple mixing of the chosen ingredients. Homogenisation may, for example, be used at various stages of mixing.

For example, a basic composition may be manufactured by a method comprising mixing a solvent, a thickening polymer and a coolant which is a cyclic carboxamide. Optionally, further ingredients may be added.

In a preferred embodiment, the method comprises mixing a solvent, a thickening polymer, a preservative, a pH adjuster, and a coolant which is a cyclic carboxamide. Optionally, the method further comprises a step of adding humectant. Optionally, the method also further comprises adding a sweetener. A preferred manufacturing process comprises the steps of providing a solvent, which is preferably water; adding humectant and preservative to the solvent and mixing to homogeneity; adding pH adjuster; adding thickening polymer; mixing to homogeneity; adding a cyclic carboxamide coolant, which is preferably N-ethyl-p-menthane-3-carboxamide; optionally adding a sweetener; mixing; and adjusting the pH. Suitably, the pH is adjusted to a pH of from pH3.8 to pH4.8. As the skilled person will understand, the mixing can be done in any suitable vessel, such as a mixing tank. Preferably, the pH adjuster is added in the form of a solution in a suitable solvent.

The following Example is intended to illustrate the invention.

### Example 1

A particularly preferred composition comprises:

| Ingredient function | Ingredient | Amount (%w/w) |
|---|---|---|
| Solvent | Water | 87.225 |
| Humectant | Propylene glycol | 10.000 |
| Thickener | Hydroxyethylcellulose | 2.000 |
| Coolant | N-ethyl-p-menthane-3-carboxamide | 0.016 - 0.020 |
| pH adjuster | Sodium hydroxide | 0.025 |
| Preservative | Benzoic acid | 0.100 |

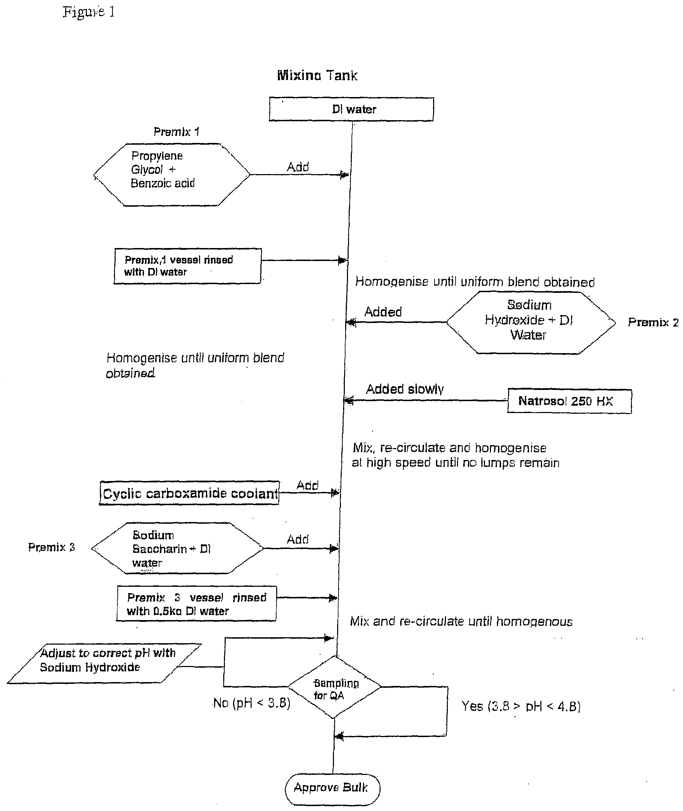

The composition was prepared according to the following method, which is illustrated in Figure 1. Water is provided in a mixing tank. Propylene glycol and benzoic acid are prepared as a first premix, and the first premix is added to the tank. The first premix vessel is rinsed with water, which is added to the tank. The contents of the tank are mixed by homogenising until a uniform blend is obtained. Sodium hydroxide and water are mixed, to form a second premix. The second premix is added to the tank, and the tank contents are homogenised to uniformity. Hydroxyethylcellulose is added slowly to the tank, with mixing, until no lumps remain. N-ethyl-p-menthane-3-carboxamide is added, followed by sodium saccharin, preferably in the form of a solution. The bulk mixture is mixed and re-circulated until homogenous, and sampling is carried out to ensure the pH is from pH 3.8 to pH 4.8. If necessary, the pH is adjusted by addition of further sodium hydroxide.

## Claims

1. Use of an aqueous condom-compatible sexual lubricant composition to provide lubrication during sexual activity, wherein the composition comprises a coolant which is a cyclic carboxamide.

2. A use according to claim 1, wherein the composition comprises a pH adjuster in an amount such that the pH of the composition is 3.8 to 4.8.

3. A use according to claim 1 or 2, wherein the composition is substantially free from menthol.

4. A use according to claim 3, wherein the composition is completely free from menthol.

5. A use according to any preceding claim, wherein the cyclic carboxamide is N-ethyl-p-menthane-3-carboxamide.

6. A use according to any preceding claim, wherein the composition comprises a second coolant.

7. A use according to claim 6, wherein the second coolant is selected from menthyl lactate, menthone glycerine acetal, menthoxy propane diol and isopulegol.

8. A use according to any preceding claim, wherein the total coolant concentration in the composition is from 0.01 to 1.5% w/w by weight of the total composition.

9. A use according to any preceding claim, wherein the concentration of cyclic carboxamide in the composition is from 0.01 to 0.3% w/w by weight of the total composition.

10. A use according to claim 9, wherein the concentration of cyclic carboxamide in the composition is from 0.016 to 0.020% w/w by weight of the total composition.

11. A use according to any preceding claim, wherein the composition comprises a solvent comprising water in combination with glycerol and/or propylene glycol.

12. A use according to any preceding claim, wherein the composition comprises a thickening polymer.

13. A use according to claim 12, wherein the thickening polymer is a cellulose.

14. A use according to claim 12 or 13, wherein the composition comprises a humectant.

15. A use according to claim 14, wherein the humectant is glycerine, a polyol or a glycol.

16. A use according to any preceding claim, wherein the composition comprises the following ingredients in amounts within the ranges indicated:
| Ingredient | Amount (% w/w) |
|---|---|
| Solvent | 75 - 90 |
| Humectant | 5 - 20 |
| Thickener | 1.5 - 2.5 |
| Cyclic carboxamide | 0.01 - 1.5 |
| pH adjuster | To give final pH from pH 3.8 to 4.8 |
| Preservative | 0.1 - 0.2 |
| Sweetener | 0.05 - 0.10 |

## Patentansprüche

1. Verwendung einer wässrigen kondomkompatiblen Sexualgleitmittelzusammensetzung, um ein Gleiten während sexueller Aktivität bereitzustellen, wobei die Zusammensetzung ein Kühlmittel umfasst, das ein cyclisches Carboxamid ist.

2. Verwendung nach Anspruch 1, wobei die Zusammensetzung einen pH-Einstellmittel in einer solchen Menge umfasst, dass der pH-Wert der Zusammensetzung 3,8 bis 4,8 beträgt.

3. Verwendung nach Anspruch 1 oder 2, wobei die Zusammensetzung im Wesentlichen frei von Menthol ist.

4. Verwendung nach Anspruch 3, wobei die Zusammensetzung vollständig frei von Menthol ist.

5. Verwendung nach einem der vorhergehenden Ansprüche, wobei das cyclische Carboxamid n-Ethyl-p-menthan-3-carboxamid ist.

6. Verwendung nach einem der vorhergehenden Ansprüche, wobei die Zusammensetzung ein zweites Kühlmittel umfasst.

7. Verwendung nach Anspruch 6, wobei das zweite Kühlmittel ausgewählt ist aus Menthyllactat, Menthonglycerinacetal, Menthoxypropandiol und Isopulegol.

8. Verwendung nach einem der vorhergehenden Ansprüche, wobei die Gesamtkonzentration des Kühlmittels in der Zusammensetzung 0,01 bis 1,5 Gew.-% der Gesamtzusammensetzung beträgt.

9. Verwendung nach einem der vorhergehenden Ansprüche, wobei die Konzentration des cyclischen Carboxamids in der Zusammensetzung 0,01 bis 0,3 Gew.-% der Gesamtzusammensetzung beträgt.

10. Verwendung nach Anspruch 9, wobei die Konzentration des cyclischen Carboxamids in der Zusammensetzung 0,016 bis 0,020 Gew.-% der Gesamtzusammensetzung beträgt.

11. Verwendung nach einem der vorhergehenden Ansprüche, wobei die Zusammensetzung ein Lösungsmittel umfasst, das Wasser in Kombination mit Glycerin und/oder Propylenglycol umfasst.

12. Verwendung nach einem der vorhergehenden Ansprüche, wobei die Zusammensetzung ein Verdickungspolymer umfasst.

13. Verwendung nach Anspruch 12, wobei das Verdickungspolymer eine Cellulose ist.

14. Verwendung nach Anspruch 12 oder 13, wobei die Zusammensetzung ein Feuchthaltemittel umfasst.

15. Verwendung nach Anspruch 14, wobei das Feuchthaltemittel Glycerin, ein Polyol oder ein Glycol ist.

16. Verwendung nach einem der vorhergehenden Ansprüche, wobei die Zusammensetzung die folgenden Inhaltsstoffe in Mengen innerhalb der angegebenen Bereiche umfasst:
| Inhaltsstoff | Menge (Gew.-%) |
|---|---|
| Lösungsmittel | 75 bis 90 |
| Feuchthaltemittel | 5 bis 20 |
| Verdickungsmittel | 1,5 bis 2,5 |
| Cyclisches Carboxamid | 0,01 bis 1,5 |
| pH-Einstellmittel | Zur Erzielung eines endgültigen pH-Werts von 3,8 bis 4,8 |
| Konservierungsmittel | 0,1 bis 0,2 |
| Süßungsmittel | 0,05 bis 0,10 |

## Revendications

1. Utilisation d'une composition aqueuse lubrifiante sexuelle compatible avec les préservatifs pour assurer la lubrification lors de l'activité sexuelle, dans laquelle la composition comprend un agent de refroidissement qui est un carboxamide cyclique.

2. Utilisation selon la revendication 1, dans laquelle la composition comprend un régulateur de pH en une quantité telle que le pH de la composition est de 3,8 à 4,8.

3. Utilisation selon la revendication 1 ou 2, dans laquelle la composition est sensiblement exempte de menthol.

4. Utilisation selon la revendication 3, dans laquelle la composition est totalement exempte de menthol.

5. Utilisation selon l'une quelconque des revendications précédentes, dans laquelle le carboxamide cyclique est le N-éthyl-p-menthane-3-carboxamide.

6. Utilisation selon l'une quelconque des revendications précédentes, dans laquelle la composition comprend un second agent de refroidissement.

7. Utilisation selon la revendication 6, dans laquelle le second agent de refroidissement est choisi parmi le lactate de menthyle, le menthone glycérine acétal, le menthoxy propane diol et l'isopulégol.

8. Utilisation selon l'une quelconque des revendications précédentes, dans laquelle la concentration totale en agent de refroidissement dans la composition est de 0,01 à 1,5 % p/p en poids de la composition totale.

9. Utilisation selon l'une quelconque des revendications précédentes, dans laquelle la concentration en carboxamide cyclique dans la composition est de 0,01 à 0,3 % p/p en poids de la composition totale.

10. Utilisation selon la revendication 9, dans laquelle la concentration en carboxamide cyclique dans la composition est de 0,016 à 0,020 % p/p en poids de la composition totale.

11. Utilisation selon l'une quelconque des revendications précédentes, dans laquelle la composition comprend un solvant comprenant de l'eau en combinaison avec du glycérol et/ou du propylène glycol.

12. Utilisation selon l'une quelconque des revendications précédentes, dans laquelle la composition comprend un polymère épaississant.

13. Utilisation selon la revendication 12, dans laquelle le polymère épaississant est une cellulose.

14. Utilisation selon la revendication 12 ou 13, dans laquelle la composition comprend un humectant.

15. Utilisation selon la revendication 14, dans laquelle l'humectant est la glycérine, un polyol ou un glycol.

16. Utilisation selon l'une quelconque des revendications précédentes, dans laquelle la composition comprend les ingrédients suivants en des quantités comprises dans les plages indiquées :
| Ingrédient | Quantité (% p/p) |
|---|---|
| Solvant | 75 - 90 |
| Humectant | 5 - 20 |
| Épaississant | 1,5 - 2,5 |
| Carboxamide cyclique | 0,01 - 1,5 |
| Régulateur de pH | Pour donner un pH final de 3,8 à 4,8 |
| Conservateur | 0,1 - 0,2 |
| Édulcorant | 0,05 - 0,10 |
